# NEW EUROPEAN PATENT SPECIFICATION

(11) **EP 0 284 240 B2**
(45) Date of publication and mention of the opposition decision: **16.06.1993**
(45) Mention of the grant of the patent: 25.07.1990
(21) Application number: 88302010.9
(22) Date of filing: 08.03.1988
(51) Int. Cl.: G03C 7/38, C07D 487/04

(54) **Photographic silver halide materials and process comprising a pyrazolotriazole coupler**
Photographische Silberhalogenidmaterialien und Verfahren, das einen Pyrazoloazolkuppler enthält
Matériaux photographiques à l'halogénure d'argent et procédé comprenant un coupleur pyrazoloazole

(30) Priority: 09.03.1987 US 23519
(43) Date of publication of application: 28.09.1988
(73) Proprietor: EASTMAN KODAK COMPANY (a New Jersey corporation), Rochester, New York 14650 (US)
(72) Inventor: Bowne, Arlyce Tolman c/o EASTMAN KODAK COMPANY, Rochester New York 14650 (US); Romanet, Robert Fogg c/o EASTMAN KODAK COMPANY, Rochester New York 14650 (US); Normandin, Sharon Eileen c/o EASTMAN KODAK COMPANY, Rochester New York 14650 (US)
(74) Representative: Baron, Paul Alexander Clifford

(56) References cited:
- EP-A- 0 176 804
- EP-A- 0 231 832
- EP-A- 0 240 852
- GB-A- 2 178 551
- JP-A-61 249 053
- JP-A-62 005 234

## Description

This invention relates to novel pyrazolotriazole couplers and to photographic silver halide materials and processes using such couplers.

Color images are customarily obtained in the photographic art by reaction between the oxidation product of a silver halide color developing agent and a dye-forming coupler. Pyrazolone couplers are useful for forming magenta dye images. Pyrazolotriazole couplers represent another class of couplers that are useful for this purpose. Examples of pyrazolotriazole couplers are disclosed in, for instance, U.S. Patent4,443,536; U.K. Patents 1,247,493; 1,252,418 and 1,398,979. An example of such a coupler is represented by the formula C-1:

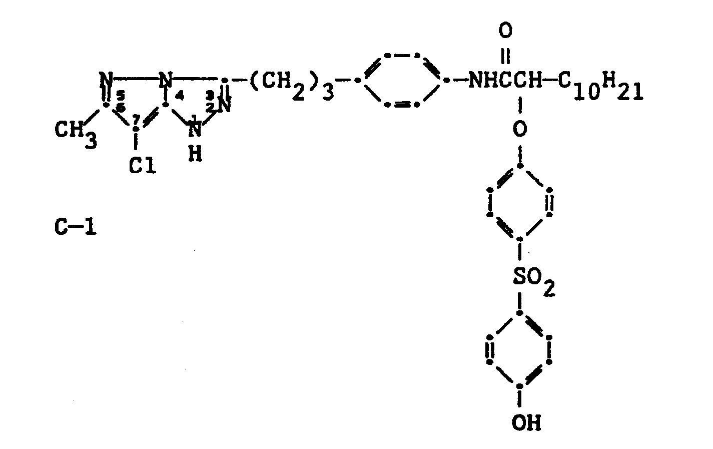

While such magenta dye-forming couplers are useful in photographic silver halide materials and processes, many such couplers do not have the desired degree of reactivity. Sufficient reactivity herein means the ability of the coupler to form the concentration of magenta dye that is desired and to enable the formation of desired maximum dye image density upon oxidative coupling. It has been desired to provide pyrazolotriazole couplers that have increased reactivity overthose described in the patent cited above and provide magenta dyes having desired hue and that do not move to other locations in the photographic element containing them, that is provide magenta dyes that are substantially immobile.

According to the present invention photographic silver halide materials are provided comprising a support bearing a photographic silver halide emulsion layer having in or adjacent the emulsion layer a novel immobile dye-forming pyrazolotriazole coupler wherein the coupler has an aliphatic or aromatic ballast group attached to the pyrazolotriazole nucleous by a secondary carbon atom containing a combination of an ether group (-O-) that enables desired magenta hue and a carboxy group (-COOH) enabling increased coupler reactivity. Such an aliphatic or aromatic ballast group typically has 14 to 40 carbon atoms. The pyrazolotriazole is capable of forming an immobile dye in a gelatino silver halide emulsion.

Such a pyrazolotriazole is, for example, a 1 H-pyrazolo-[3,2-c]-s-triazole or a 1 H-pyrazolo[2,3-b]-s-triazole. A 1 H-pyrazolo[2,3-b]-s-triazole herein can also be named as a 1 H-pyrazolo[1,5-b]-1,2,4-triazole. The latter nomenclature has been used in the photographic art in, for example, U.S. Patent 4,540,654.

It has been found that the combination of groups including one carboxy group on the ballast group provides the desired degree of reactivity. It is surprising that the presence of the carboxy group on a ballast group of a pyrazolotriazole coupler enables the desired magenta hue and increased reactivity without adversely affecting the immobility of the resulting image dye.

Any ballast group as described containing a combination of an ether group and a carboxy group is useful on the pyrazolotriazole coupler. The ballast group contains a sufficient number of carbon atoms to enable the dye formed from the coupler to be immobile in a gelatino silver halide emulsion. The ballast group for this purpose typically contains 14 to 40 carbon atoms.

A preferred ballast group as described comprises a secondary carbon atom bonded directly to the pyrazolotriazole nucleus and the ether moiety bonded directly to this secondary carbon atom.

An illustrative ballast group is represented by the following formula: 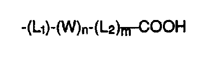 wherein,
L₁ is selected from unsubstituted aliphatic or ortho substituted arylene groups attached to the pyrazolotriazole nucleous by a secondary carbon atom;
L₂ is unsubstituted or substituted alkylene or arylene.
W represents a divalent functional group selected from alkylene, arylene, 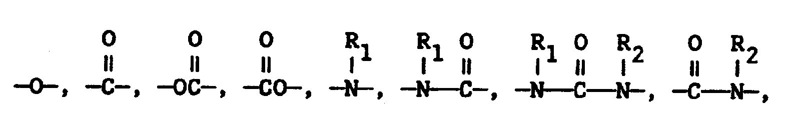 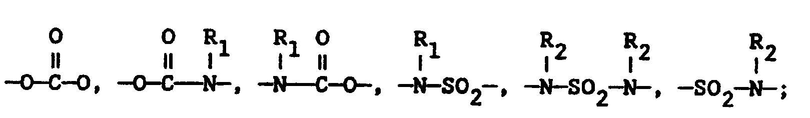 wherein
R₁ and R₂ are individually hydrogen, unsubstituted or substituted alkyl, aryl, alicyclic, or heterocyclic groups; n and m are individually zero or a positive integer, preferably 1, 2, or 3, provided that,
at each occurrence, the individual L (or W) group may be the same as or different from the other L (or W) groups; and,
at least one of L₁, W and L₂ comprises an ether (-O-) group that enables a magenta dye hue.

Preferably, at least one of L₁, W and L₂ comprises at least one sulfonamido group.

The total number of carbon atoms in the carboxy-substituted ballast group is typically within the range of 14 to 40.

The aliphatic ballast group as described is typically substituted alkylene, such as alkylene containing 14 to 40 carbon atoms.

The ballast group is, as described, additionally optionally substituted with groups that do not adversely affect the coupler or the dye formed from the coupler. The ballast group can, for example, be optionally substituted with, halogen, such as chlorine, bromine and fluorine; alkyl, such as alkyl containing 1 to 30 carbon atoms, for example, methyl, ethyl, propyl, t-butyl, n-butyl and eicosyl; cycloalkyl, such as cyclohexyl and cyclopentyl; alkoxy, such as alkoxy containing 1 to 20 carbon atoms, for example, methoxy, propoxy, butoxy and dodecy- loxy; amino, such as dioctylamino, anilino, and dimethylamino; carbonamido, such as acylamino containing 1 to 20 carbon atoms, for example acetamido, stearamido, ureido, and benzamido; alkylthio, such as alkylthio containing 1 to 20 carbon atoms, for example methylthio, ethylthio, and dodecylthio; aryl, such as aryl containing 6 to 30 carbon atoms, for example phenyl and naphthyl; aryloxy, such as aryloxy containing 6 to 20 carbon atoms, for example phenoxy and naphthoxy; cyano; nitro; arylthio, such as arylthio containing 1 to 20 carbon atoms, for example phenylthio and naphthylthio; sulfamyl, such as methylsulfamyl, butylsulfamyl and phenylsulfamyl; or a heterocyclic group comprised of atoms selected from carbon, nitrogen, oxygen and sulfur atoms necessary to complete a 5- or 6-member ring, for example a pyrrolyl, oxazolyl, or pyridyl ring.

Examples of useful ballast groups containing the carboxy group are as follows:

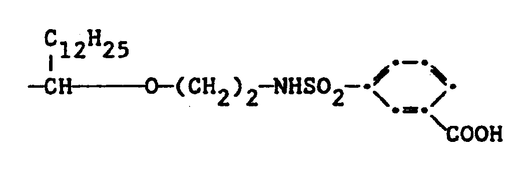

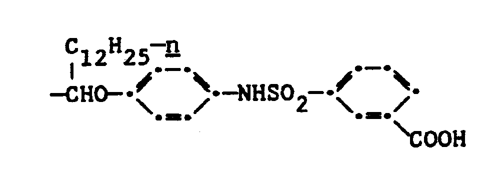

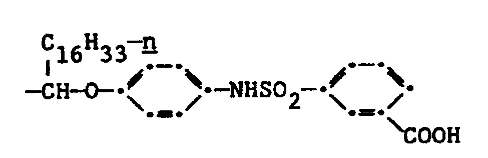

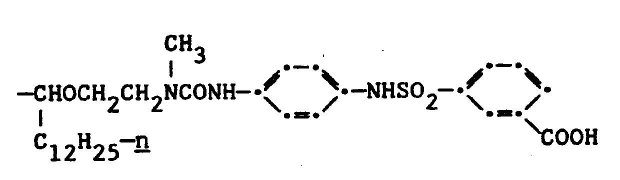

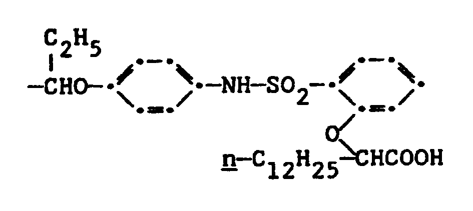

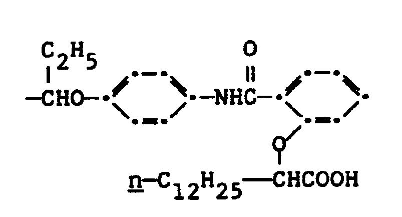

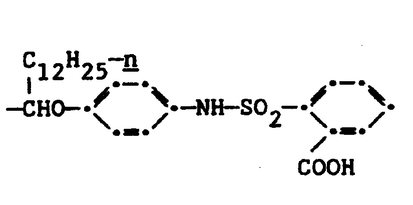

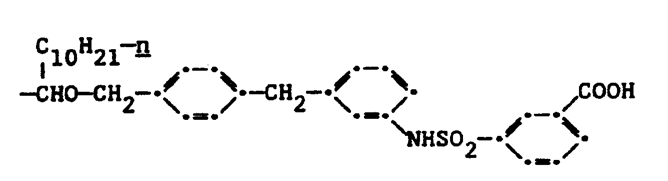

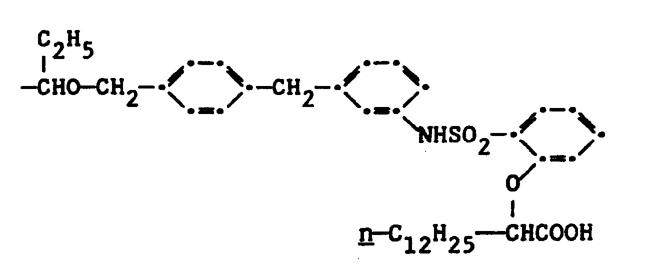

Illustrative examples of a 1 H-pyrazolo[3,2-c]-s-triazole and a 1 H-pyrazolo[2,3-b]-s-triazole are represented by formula II and formula III, respectively: 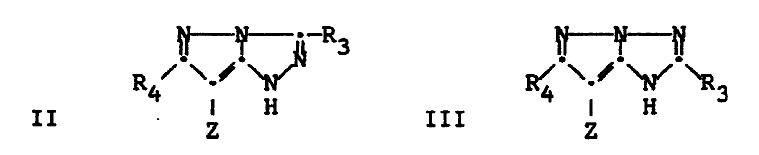 wherein
Z is hydrogen or a coupling-off group incapable of reducing silver;
R₃ and R₄ are individually hydrogen or substituent that does not adversely affect the coupler or the dye formed from the coupler, and wherein at least one of R₃ and R₄ is a ballast group, as described.

The pyrazolotriazole coupler typically contains in a position not occupied by Z or a ballast group as described, a substituent that does not adversely affect the desired properties of the coupler or the dye formed from the coupler. Such a substituent in a position not occupied by Z or a ballast group as described typically can aid such properties as solubility, diffusion resistance, dye hue and the like. For example, the pyrazolotriazole can contain in such a position, amino, such as dioctylamino, ethylhexylamino and anilino; acylamino, such as acetamido, benzamido and stearamido; ureido; carboxy; aryl, such as aryl containing 6 to 40 carbon atoms, for example, phenyl, naphthyl, carboxyphenyl; alkyl, such as alkyl containing 1 to40 carbon atoms, for example, methyl, ethyl, propyl, t-butyl, n-butyl, actyl and eicosyl; and a heterocyclic group, such as a heterocyclic group containing the atoms selected from carbon, nitrogen, oxygen and sulfur atoms necessary to form a 5- or-6 member heterocyclic group, such as pyrrolyl, oxazolyl and pyridyl. Such group in this position are unsubstituted or optionally substituted with groups that do not adversely affect the coupler or the dye formed. For example, the group can be substituted with carboxy, alkyl, such as alkyl containing 1 to 30 carbon atoms, for example methyl, ethyl, propyl, butyl and eicosyl; aryl, such as aryl containing 6 to 30 carbon atoms, for example phenyl, naphthyl and mesityl; cyano; nitro; a heterocyclic group, such as a heterocyclic group comprised of atoms selected from carbon, oxygen, nitrogen and sulfur atoms necessary to complete a 5- or 6-member ring, such as a pyrrolyl, oxazolyl, and pyridyl; or, W₁-Rₛ. Such groups are unsubstituted or optionally substituted with groups that do not adversely affect the coupler or dye formed from the coupler.

W₁ is a linking group that does not adversely affect the desired properties of the coupler and the dye formed W₁ is, for example,

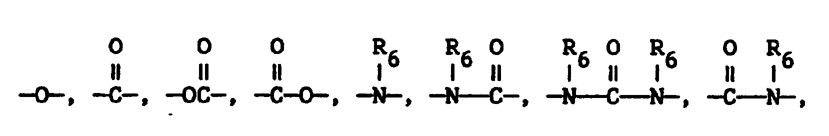

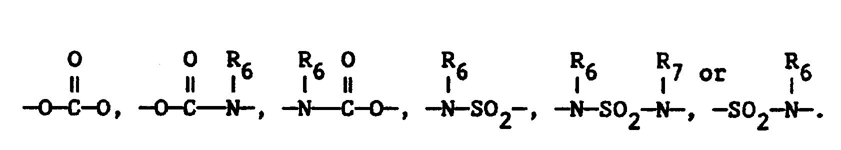

R₅ is a substituent that does not adversely affect the coupler or the dye formed, such as alkyl, for example alkyl containing 1 to 30 carbon atoms, including methyl, ethyl, propyl, t-butyl, n-butyl, octyl, and eicosyl; aryl, such as aryl containing 6 to 30 carbon atoms, for example phenyl, mesityl and naphthyl; or heterocyclic group, such as a 5- or 6-member heterocyclic group comprised of atoms selected from carbon, nitrogen, oxygen and sulfur atoms necessary to complete a 5 or 6 member heterocyclic ring, for example an oxazolyl, pyrrolyl, pyridyl or thienyl ring.

R₆ and R₇ are individually alkyl, such as alkyl containing 1 to 40 carbon atoms, for example methyl, ethyl, propyl, butyl and eicosyl; aryl, such as aryl containing 6 to 30 carbon atoms, for example phenyl, mesityl and naphthyl; or heterocyclic, such as a 5- or 6-member heterocyclic group comprised of atoms selected from carbon, oxygen, nitrogen and sulfur atoms necessary to form a 5 or 6 member ring, for example pyrrolyl, oxazolyl and pyridyl.

Examples of groups useful in other positions on the pyrazoloazole nucleus not occupied by the ballast group are as follows:
t-butyl
methyl
phenyl
2-propyl
t-octyl

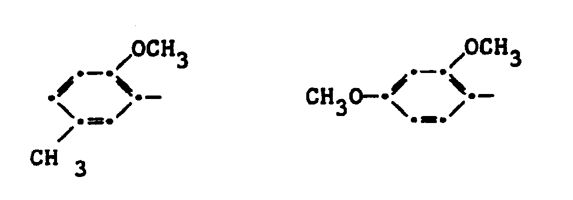

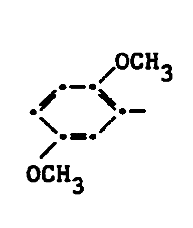

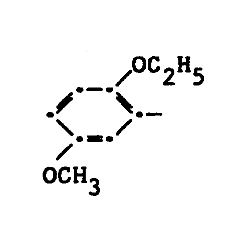

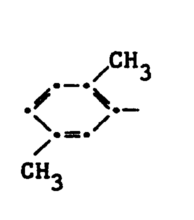

The pyrazolotriazole couplers contain in the coupling position hydrogen or a coupling-off group that is incapable of reducing silver. For example, a 1 H-pyrazolo[3,2-c]-s-triazole as described contains in the 7-position hydrogen or a coupling-off group that is incapable of reducing silver.

Coupling-off groups, defined by Z herein, are well known to those skilled in the photographic art. Such groups can determine the equivalency of the coupler, can modify the reactivity of the coupler, or can advantageously affect the layer in which the coupler is coated or other layers in the element by performing, after release from the coupler, such functions as development inhibition, bleach acceleration, color correction, development acceleration and the like. It is important that the coupling-off group be incapable of reducing silver in orderthat the coupling-off group not cause undesirable changes in image formation. Representative classes of coupling-off groups include halogen, alkoxy, aryloxy, heterocyclyloxy, sulfonyloxy, acyloxy, carbonamido, imido, heterocyclic, thiocyano, alkylthio, arylthio, heterocyclylthio, sulfonamido, phosphonyloxy and arylazo. They are described in, for example, U.S. Patents 2,355,169; 3,227,551; 3,432,521; 3,476,563; 3,617,291; 3,880,661; 4,052,212 and 4,134,766; and in U.K. Patents and published patent applications 1,466,728; 1,531,927; 1,533,039; 2,006,755Aand 2,017,704A, the disclosures of which are incorporated herein by reference.

Examples of specific coupling-off groups are as follows: 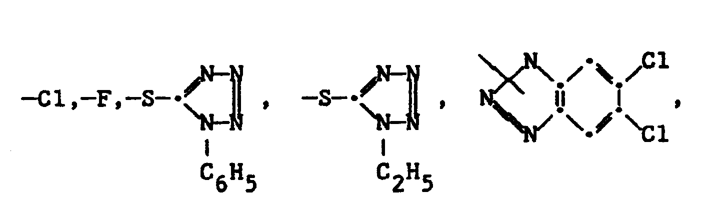
-SCN, -OCH₃, -OC₆H₅, -OCH₂CONHCH₂CH₂OH,
-OCH₂CONHCH₂CH₂OCH₃, -OCH₂CONHCH₂CH₂OCOCH₃, 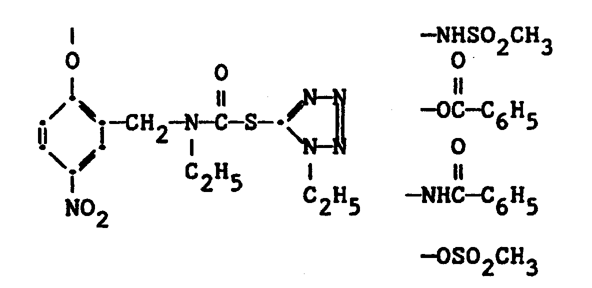 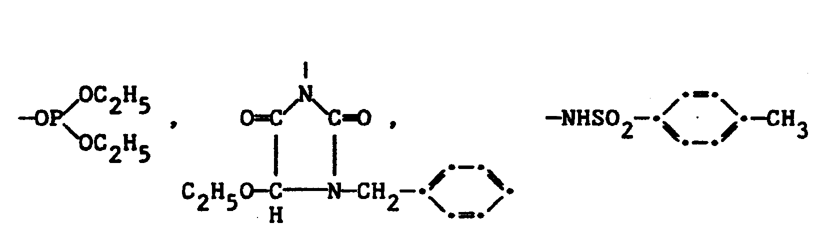 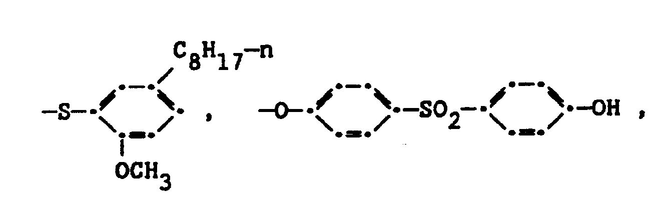
-S(̵CH₂)̵₂COOH, 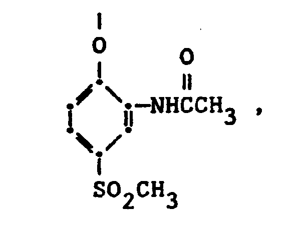
and

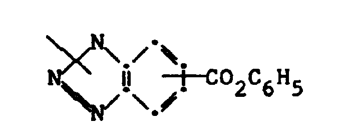

The coupling-off group as described can contain a water-solubilizing group, such as a carboxy group. The total hydrophilicity of the coupler and the dye formed from the coupler should not be high enough to cause the coupler and the dye formed to be mobile in the photographic element.

The ballast group, as described, is an organic radical of such size and configuration as to confer on the coupler molecule sufficient bulk to render the coupler immobile, that is sufficiently non-diffusible from the layer and the position in the layer in which it is coated in a photographic element. The coupler can optionally contain other ballast groups than the ballast group containing the carboxy group as described. Couplers of the invention can optionally be attached to polymeric chains through one or more of the described groups on the coupler moiety, such as the leaving group or the 2-, 3- and or 6-position substituents.

Representative substituents on such a ballast group not containing a carboxy group include alkyl, aryl, alkoxy, aryloxy, amino, carbonamido, carbamoyl, alkanesulfonyl, arenesulfonyl, sulfonamido, and sulfamoyl substituents.

Pyrazolotriazole couplers of the invention are prepared by the general method of synthesis described in Research Disclosure, August 1974, Item No. 12443 published by Kenneth Mason Publications, Ltd. The Old Harbourmaster's, 8 North Street, Emsworth, Hampshire P010 7DD, England and U.S. Patent 4,540,654.

The couplers are prepared by one of a number of routes exemplified by the following synthesis Schemes examples:
(Et herein means ethyl (C₂H₅)).
(Ac herein means acetate).
(Ac₂0 herein means acetic anhydride).
(A herein means with heating.)

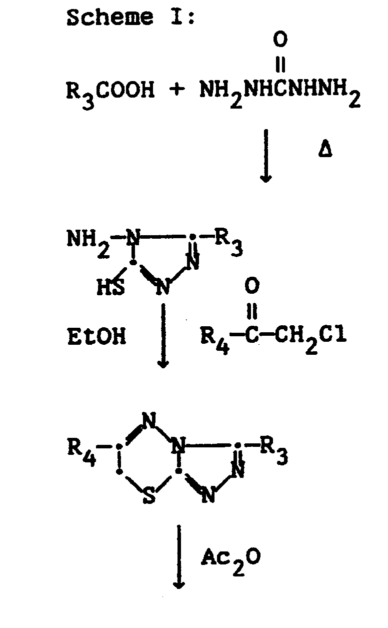

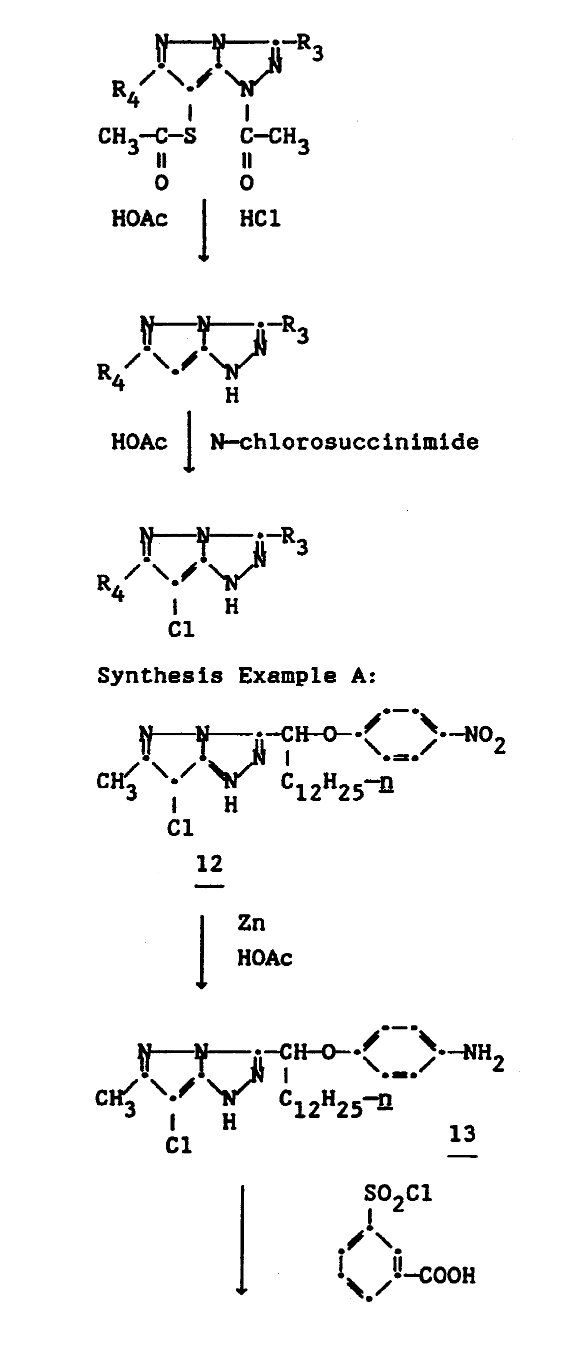

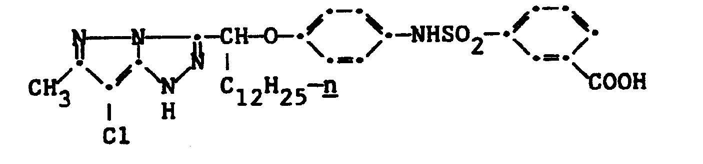

11.6 g of 12 was dissolved in 50 ml HOAc and kept at or below 20°C while 12 grams zinc dust was added portion-wise. After stirring one hour, the reaction mixture was added to water. Ethylacetate was added and the organic layer washed, dried, and evaporated to a brown gum and triturated with Et₂0 to give 13 (8.3 g) as a light tan solid.

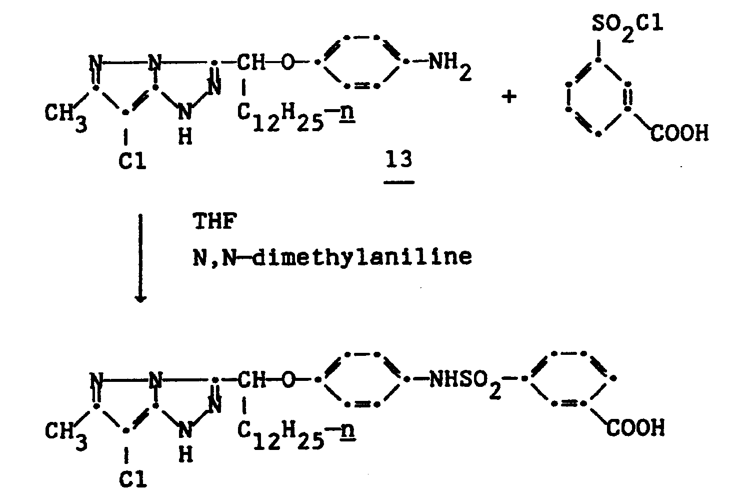

4.0 g (.0088 mol) of 13 were suspended in 40 ml THF and 2.0 g (.0090 mol) sulfonyl chloride were added followed by 2.4 ml (.018 mol) N,N-dimethylaniline. After stirring one hour the reaction mixture was added to dilute HCI and extracted with ether. The ether extracts were washed with water, dried and evaporated to give 5.2 g crude oil. The product was chromatographed on silica gel and crystallized from acetonitrile to give 2.8 g of the desired product as a white solid.
Elemental Analysis:
Theoretical:
C: 59.1; H:6.4; N: 11.1
Found:
C: 59.0; H: 6:3; N: 11.1.
NMR Analysis
8: .90 (t,3H); .93-1.55 (m,20H); 2.00-2.4 (m,s,5H); 5.25 (t,1 H); 6.75 (d,2H); 6.85 (d,2H); 7,38 (t,1 H); 7.72 (d,1 H); 8.07 (d,1 H); 8.32 (s,1 H); 8.98 (s,1 H).

### Synthesis Example B:

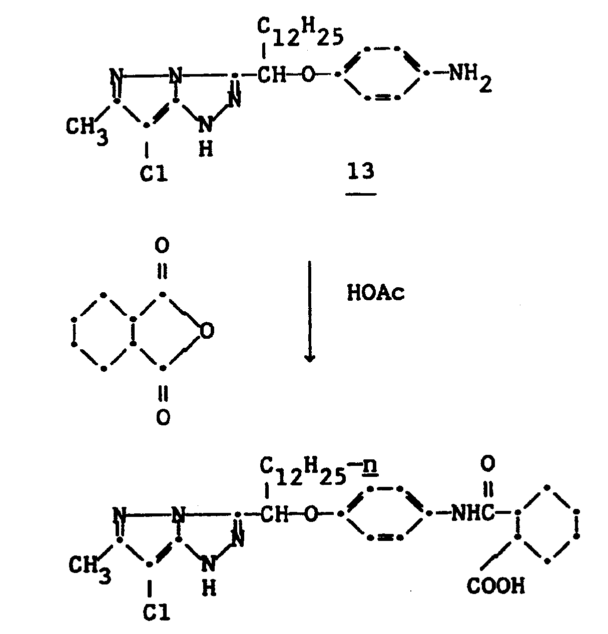

4.0 g of 13 were dissolved in acetic acid and 1.28 g anhydride added. After stirring one half hour the mixture was added to water and extracted with ethyl acetate. The organic layer was washed with water, dried and evaporated. After chromatography in silica gel, 2.5 g white solid was obtained, (mp 146 dec.).
Elemental Analysis:
Theoretical:
C: 64.1; H: 7.6; N: 11.7
Found:
C: 63.9; H: 7.5; N: 11.7
'H NMR: 80.8-3.0 (m,38H); 5.4 (t,1H); 6.9 (d,2H); 7.5 (d,2H); 9.1 (s, 1H).

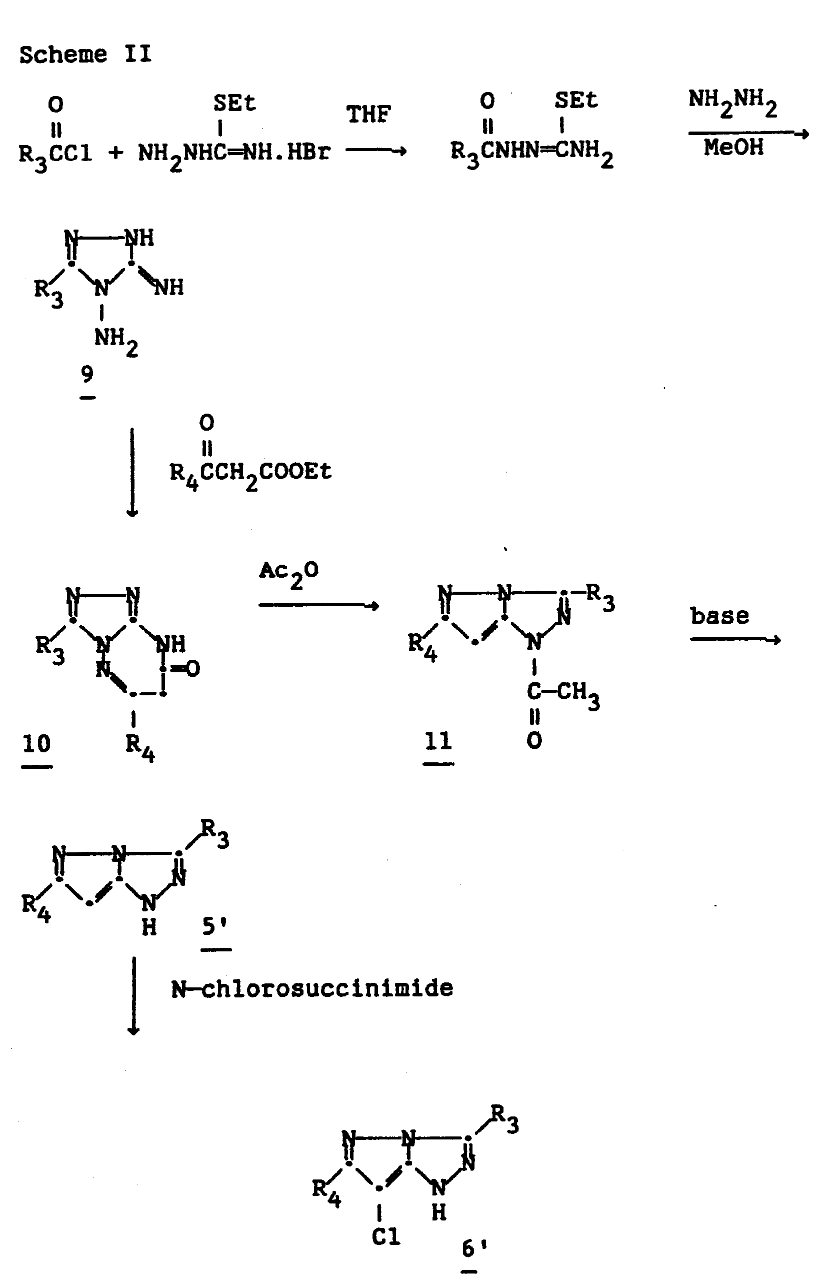

### Synthesis Example C:

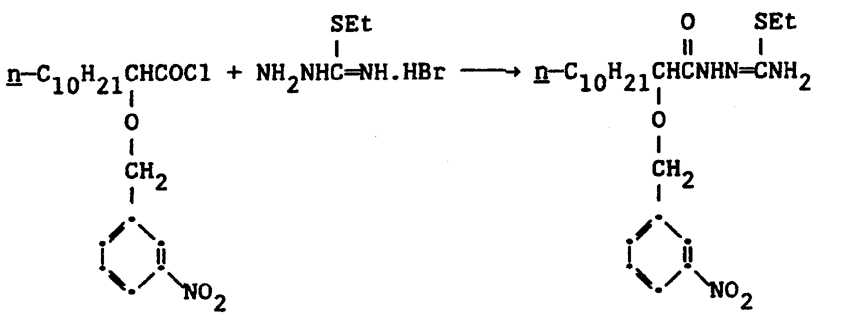

134.6 g (.364 mol) of above acid chloride and 73 g (.364 mol) of the above hydrobromide salt were stirred in THF for 2 hours. A complete solution was not obtained. The solvent was evaporated off and the resulting paste stirred in ligroin. The solid was filtered to give 180 g pasty solid. The solid dissolved in 600 ml ethanol and 600 ml water, and 42 ml conc. NH₄0H added slowly followed by 3500 ml cold water. After stirring 2.5 hours, a solid was filtered off and crystallized from CH₃CN to give 85 g white solid.

Elemental Analysis:
Theoretical:
58.4; H: 8.0; N: 12.4; S: 7.1.
Found::
58.4; H: 7.9; N: 12.5; S: 7.1.
NMRAnalysis:

'H NMR: 8 .80-.90 (t,3H); 1.10-1.59 (m,16H); 1.75-2.70 (m,2H); 4.32-4.75 (m,4H); 5.0 (s,2H); 5.65-6.00 (s,1 H); 7.4 (t,1 H); 7.64 (d,1 H); 8.0 (d,1 H); 8.1 (s,1 H).

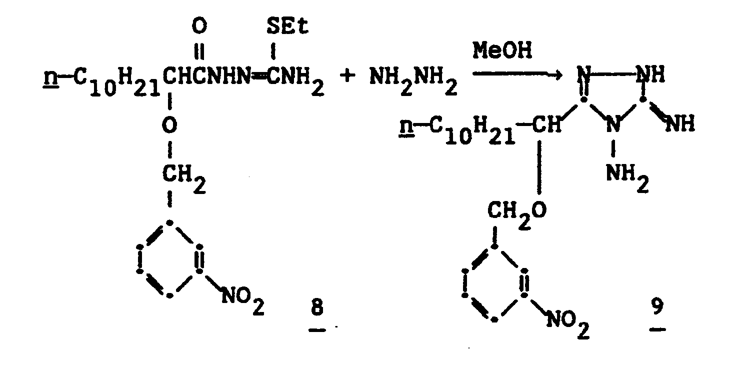

9.2 g (.288 mol) hydrazine were added to a suspension of 87 g (.192 mol) of 8 in 230 ml methanol and refluxed 3.5 hours. Most of the methanol was evaporated and the residue treated twice with 500 ml water, mixed several minutes, and the aqueous layer decanted off. The residue was dissolved in 800 ml THF and dried with MgS0₄ and concentrated to a brown oil. The product was crystallized from CH₃CN to give 46 g of cream colored solid.

Elemental Analysis:
Theoretical:
59.4; H: 8.0; N: 20.8;
Found:
59.2; H: 7.7; N: 20.6.

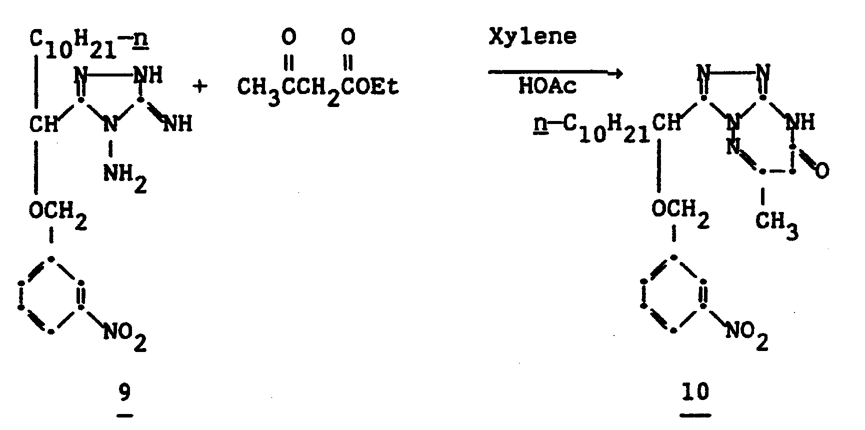

42.5 g (.105 mol) of 9 was heated 4 hours on a steam bath with 40 ml ethyl acetoacetate and 20 ml xylene and 6 ml acetic acid. After cooling the product was dissolved in CH₂CI₂/EtOAc and chromatographed on silica gel changing to straight EtOH to elute the product. The product was crystallized from CH₃CN to give 20 g solid. Elemental Analysis:
Theoretical:
61.3; H: 7.3; N: 17.9;
Found:

61.2; H: 7.3; N: 17.9

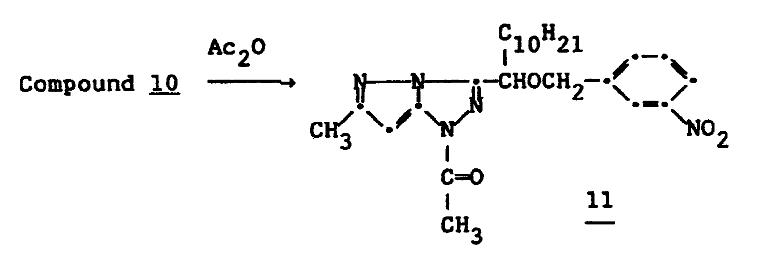

40 g (.084 mol) of 10 were refluxed one hour in 114 ml acetic anhydride. The product was poured into 31 water with rapid stirring and stirred 1 1/2 hours. EtOAc was added and the organic layer washed, dried and evaporated to brown oil. The product was chromatographed on silica gel with CH₂CI₂ to give 30 g white solid. NMRAnalysis:
'H NMR: 8 .85 (t,3H); 1.10-1.70 (m,16H); 2.05-2.31 (m,2H); 2.35 (s,3H); 2.60 (s,3H); 4.70 (s,2H); 4.75 (t,1H); 6.05 (s,1 H); 7.45 (t,1 H); 7.65 (d,1 H); 8.05 (d,1 H); 8.22 (s,1 H). 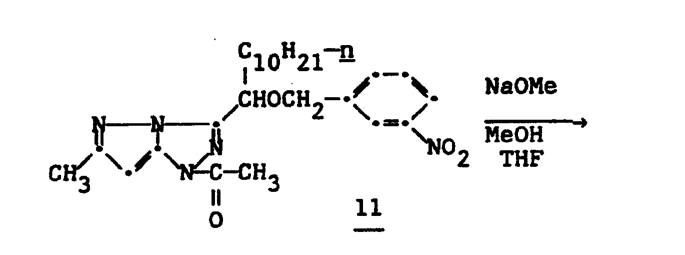 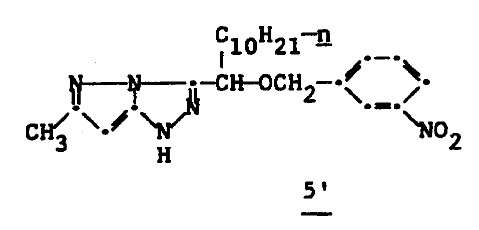
28 g (.059 mol) 11 was dissolved in 50 ml THF and 50 ml methanol. 3.8 g sodium methylate was added portion-wise, stirred 15 minutes and added to dilute HCI and extracted with EtOAc. The product was dried with MgS0₄ and concentrated to a light yellow solid. The product was triturated with ligroin and filtered to provide 25 g of solid.

Compound 5' was converted to compound 6' having a chlorine atom in the 7-position in the same manner as compound 5 was converted to compound 6.

Elemental Analysis:
Theoretical:
C: 59.8; H: 7.0; N: 15.2
Found:
C: 59.9; H: 6.8; N: 15.1
NMRAnalysis:
'H NMR: 8.87 (t,3H); 1.04-1.68 (m,16H); 1.95-2.38 (m,2H); 2.44 (s,3H); 4.59 (t,H); 7.22-7.71 (m,2H); 7.97-8.20 (m,2H).

5,0 g (.011 mol) of compound 6' was dissolved in 100 ml propionic acid and cooled to 0°. 2 ml concentrated HCI was added followed by 25 g zinc dust added portion-wise. The solvent was evaporated and the residue taken up in ethyl acetate and washed with sodium bicarbonate. The product amine was dried over magnesium sulfate and concentrated to 5.2 g orange oil.

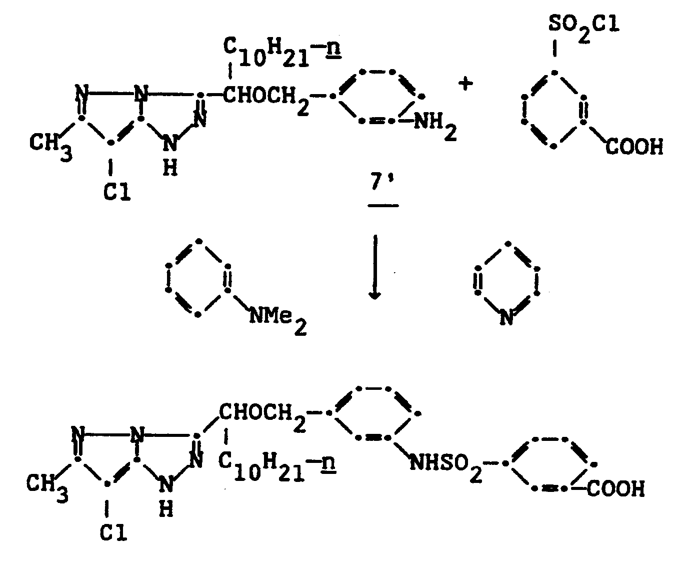

5.2 g (.011 mol) amine 7' was dissolved in 50 ml pyridine and 5 ml N,N-dimethyl aniline. 2.6 g (.012 mol) m-(chlorosulfonyl)benzoic acid was added and after one hour the reaction poured into cold dilute HCI. Ethyl acetate was added and the organic layer washed, dried and evaporated to dark glass. The product was chromatographed on silica gel to obtain, after trituration with chlorobutane, a white solid, (3,2 g) (mp 105-107°C.). Elemental Analysis: Theoretical: 15
C: 58.5; H: 6.2; N: 11.4; S: 5.2
Found:
C: 58.6; H: 6.1; N: 11.5; S: 5.4.
NMRAnalysis:
'H NMR: 8 1.0 (t,3H); 1.1-1.7 (m, 16H); 2.0-2.3 (m,2H); 2.35 (s,3H); 4.40 (d,3H); 4.60 (6,1 H); 6.90-7.30 (m,4H); 7.60 (t,1 H); 7.90-8.27 (m,2H); 8.46 (s,1 H); 10.25 (s,1 H).

### Synthesis Example D:

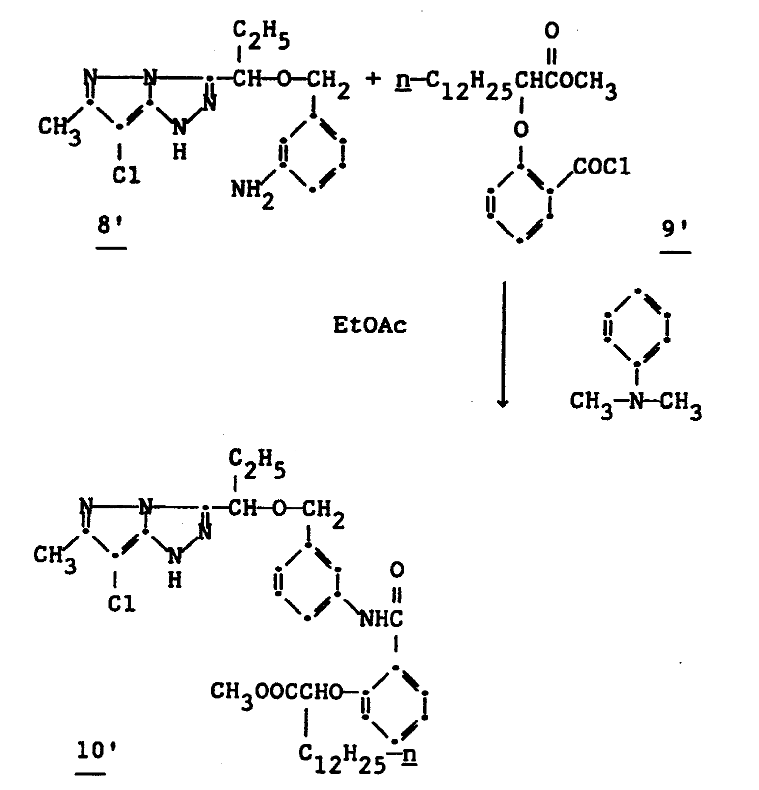

5.6 g (.0142 mol) of the ballast acid chloride 9' was added to a stirred solution of 4.5 g (.0142 mol) of the amine 8' which was synthesized in a manner analogous to the amine in Example E and 5 g (.04 mol) N,N-Dimethylaniline dissolved in 200 ml ethyl acetate. The reaction mixture was stirred at ambient temperature for one hour.

The reaction mixture was washed twice with 5% HCI solution. Dried over MgS0₄ and concentrated to brown oil. Chromatography through a column of silica gel yielded 8.5 g light yellow oil.

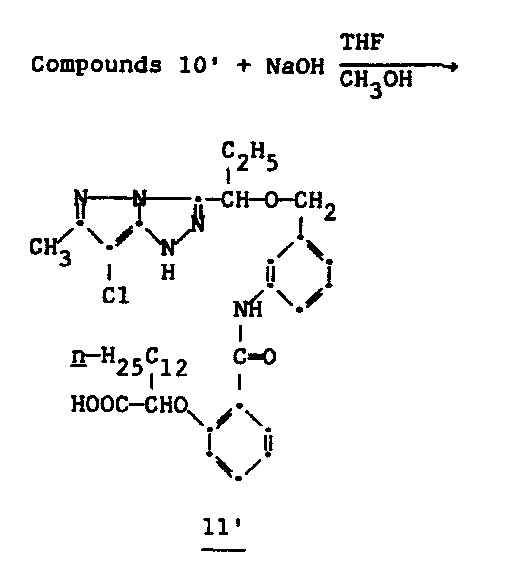

5 g of sodium hydroxide dissolved in 30 ml water was added to a stirred solution of 8.5 g of the ester 10' dissolved in 60 ml tetrahydrofuran and 40 ml methanol. The mixture was stirred at ambient temperature for 10 minutes.

The reaction mixture was poured into cold 5% HCI. The resulting oil was extracted with ethyl acetate, dried over MgS0₄ and concentrated to a glass.

Chromatography through a column of silica gel yielded 3.0 g of 11', a white solid.
Elemental Analysis:
Calculated:
N: 10.5%; C: 64.9%; H 7.3%
Found:
N: 10.5%; C: 64.8%; H 7.0%
NMRAnalysis:
0.7-1.0 (m,6H); 1.05-1.72 (m,22H); 1.95-2.25 (m,4H); 5.05-5.15 (m,1H); 6.80-7.70 (m,6H); 7.90-8.30 (m2H); 10.5 (s,1 H).

### Synthesis Example F

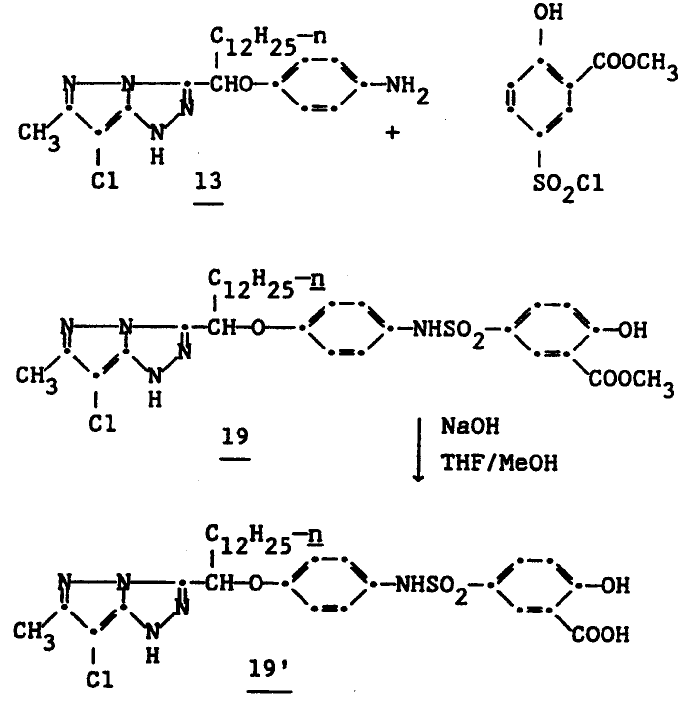

3.2 g (.0069 mol) L3 (prepared as in synthesis example A) was dissolved in 25 ml pyridine and 1.73 g sulfonylchloride was added. After stirring 1/2 hour at room temperature the reaction mixture was poured into dilute HCI and extracted with ethyl acetate. The organic layers were washed with water, dried, and evaporated, and chromatographed on silica gel. The product was dissolved in 25 ml MeOH and 25 ml THF and 5 ml 50% sodium hydroxide solution added. After stirring 20 minutes, the reaction mixture was added to dilute HCI and extracted with ethyl acetate. The organic layers were washed with water, dried, and evaporated. The product was purified by chromatography and trituration with acetonitrile to yield 1.5 g of white solid 19'.
Elemental Analysis:
Theoretical C:
57.6; H: 6.2; N: 10.8;
Found C:
57.1; H: 6.0; N: 10.7.

### Synthesis Example G

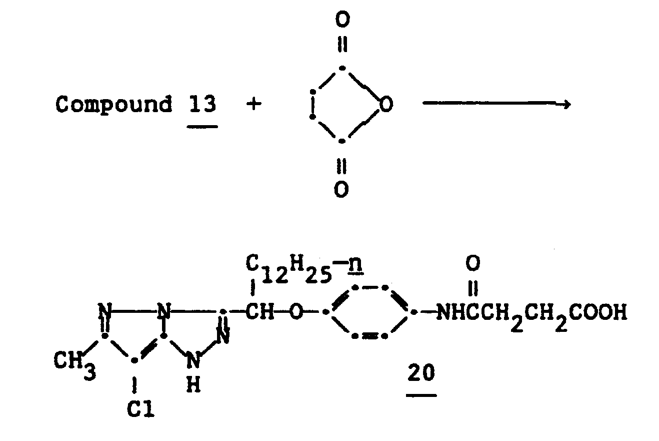

Compound 20 was prepared in the same mannerfrom compound 13 as compound 14' was prepared from compound 14.

Elemental Analysis:
Theoretical C:
61.6; H: 7.4; N: 12.8;

Found C:
60.7; H: 7.1; N: 12.6
NMRAnalysis:
'H NMR: 8 .6-1.5 (m,25H); 2.1-2.7 (m,7H); 5.5 (t,1H); 6.9 (d,2H); 7.4 (d,2H).

### Synthesis Example H

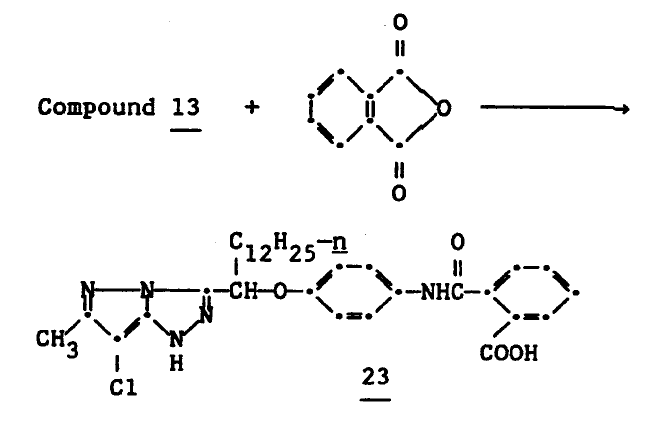

Compound 13 was prepared as in synthesis example A. It was reacted with the respective anhydride to form compound 23. The desired compound 23 was identified by Elemental Analysis and NMR Analysis: Theoretical C:
64.7; H: 6.8; N: 11.8;
Found C:
64.4; H: 6.8; N: 11.8.
NMR Analysis:
'H NMR: 8 .82 (t,3H); .85-1.60 (m,22H); 2.3 (s,3H); 5.55 (t, 1H); 6,95 (d,2H); 7.5-7.7 (m,4H); 7.90 (d,2H).

Examples of such compounds that can be prepared by this method are as follows:

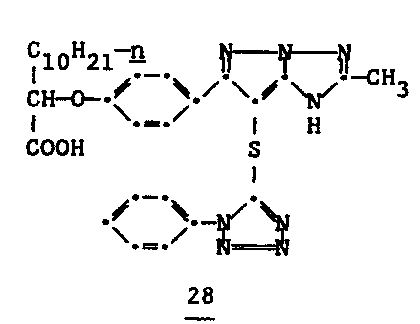

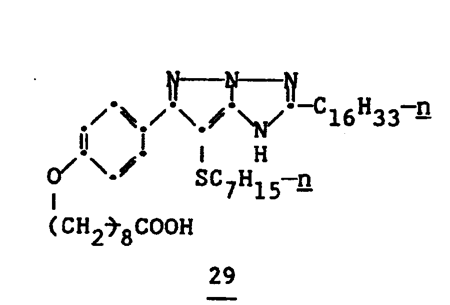

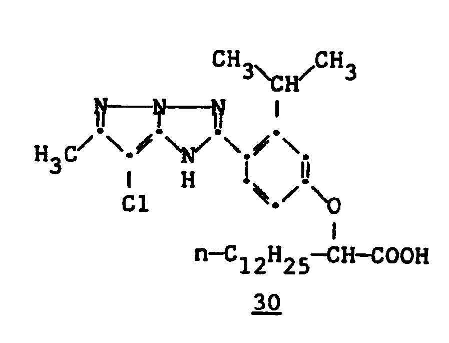

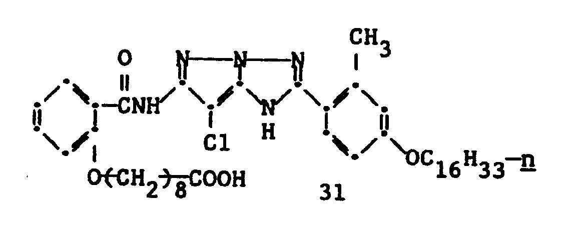

The pyrazolotriazole couplers of the invention can be used for purposes and in ways in photographic materials in which pyrazoloazole couplers are known to be useful in the photographic art.

The photographic silver halide materials can be single color elements or multicolor elements. In a multicolor element, the dye-forming pyrazolotriazole coupler as described is typically associated with a green-sensitized emulsion, although it can be associated with an unsensitized emulsion or an emulsion sensitized to a different region of the spectrum. Multicolor elements typically contain dye image-forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art. In an alternative format, the emulsion sensitive to each of the three primary regions of the spectrum can be disposed as a single segmented layer.

Atypical multicolor photographic element comprises a support bearing a cyan dye image-forming unit comprised of at least one red-sensitive silver halide emulsion layer having associated therewith at least one cyan dye-forming coupler, a magenta dye image-forming unit comprising at least one green-sensitive silver halide emulsion layer having associated therewith at least one magenta dye-forming coupler and a yellow dye image-forming unit comprising at least one blue-sensitive silver halide emulsion layer having associated therewith at least one yellow dye-forming coupler. The element can contain additional layers, such as filter layers, interlayers, overcoat layers, subbing layers, and the like.

In the following discussion of useful materials for use in the emulsions and elements of this invention, reference will be made to ResearchDisclosure, December 1978, Item No. 17643. This publication will be identified hereafter by the term "Research Disclosure".

The silver halide emulsions employed in the elements of this invention can be either negative-working or positive-working. Suitable emulsions and their preparation are described in Research Disclosure Sections I and II and the publications cited therein. Suitable vehicles for the emulsion layers of elements of this invention are described in Research Disclosure Section IX and the publications cited therein.

The elements of the invention can include additional couplers as described in Research Disclosure Section VII, paragraphs D, E, F and G and the publications cited therein. These couplers can be incorporated in the elements and emulsion as described in Research Disclosures of Section VII, paragraph C and the publications cited therein.

The photographic elements of this invention or individual layers thereof, can contain, for example, brighteners (see Research Disclosure Section V), antifoggants and stabilizers (See ResearchDisclosure Section VI), antistain agents and image dye stabilizers (see Research Disclosure Section VII, paragraphs I and J), light absorbing and scattering materials) see Research Disclosure Section VIII), hardeners (see Research Disclosure Section XI), plasticizers and lubricants (see Research Disclosure Section XIII), matting agents (see ResearchDisclosure Section XVI) and development modifiers (see Research Disclosure Section XXI) colored masking couplers, bleach accelerators and competing couplers.

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the element with a color developing agent to reduce developable silver halide and oxidize the color developing agent. Oxidized color developing agent in turn reacts with the coupler to yield a dye.

Preferred color developing agents are p-phenylenediamines. Especially preferred are
4-amino-3-methyl-N,N-diethylaniline hydrochloride,
4-amino-3-methyl-N-ethyl-N-(3-(methanesulfonamido)ethylaniline sulfate hydrate,
4-amino-3-methyl-N-ethyl-N-(3-hydroxyethylaniline sulfate,
4-amino-3-(3-(methanesulfonamido)ethyl-N,N-diethylaniline hydrochloride and
4-amino-N-ethyl-N-(2-methoxyethyl)-m-toluidine-di-p-toluenesulfonic acid.

With negative working silver halide this processing step leads to a negative image. To obtain a positive (or reversal) image, this step can be preceded by development with a non-chromogenic developing agent to develop exposed silver halide, but not form dye, and then uniform fogging of the element to render unexposed silver halide developable. Alternatively, a direct positive emulsion can be employed to obtain a positive image.

Development is followed by the conventional steps of bleaching, fixing, or bleach-fixing, to remove silver and silver halide, washing and drying.

The following examples further illustrate the invention.

### Examples 1 - 21

Photographic elements were prepared by coating a cellulose acetate-butyrate film support with a photosensitive layer containing a silver bromoiodide emulsion at 0.91 g Ag/m², gelatin at 3,77 g/m², and one of the couplers designated in the following Table I dispersed in half its weight at tricresyl phosphate and coated at 1.62 mmole/m². The photosensitive layer was overcoated with a layer containing gelatin at 1.08 g/m² and the bis-vinyl-sulfonylmethyl ether at 1.75 weight percent based on total gelatin.

Samples of each element were imagewise exposed through a graduated-density test object and processed at40°C employing the following color development solution, then stopped, bleached, fixed, washed, and dried to produce stepped magenta dye images.

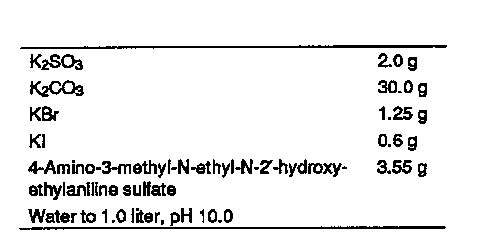

Sensitometry of these processed elements provided the maximum density (Dmax) and relative gamma (G) values presented in the following tables. The G value in each case was obtained by dividing the gamma (contrast) of each sample by that of control coupler C-9 processed in the same set.

Coupler C-9 has the structure:

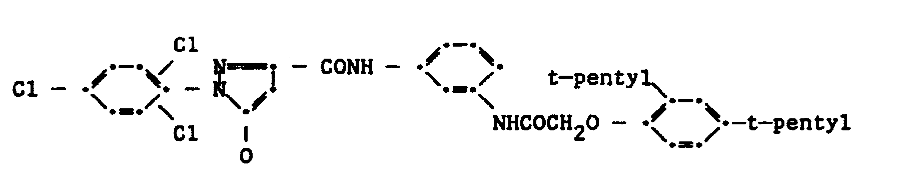

It can be seen from the data that coupler activity evidenced by the Dmax and especially the G values is generally higher for compounds of the invention than forthe comparison compounds (C-numbers in the tables).

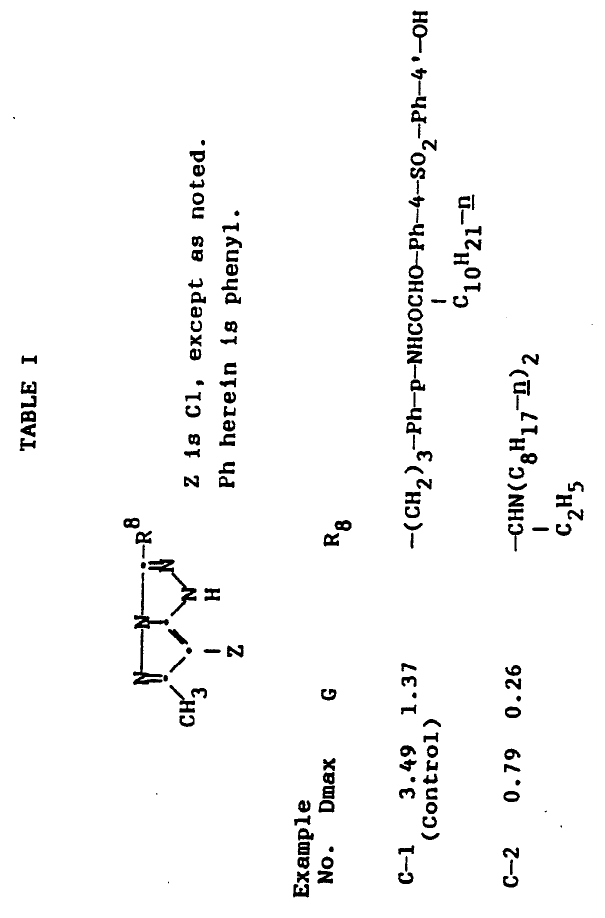

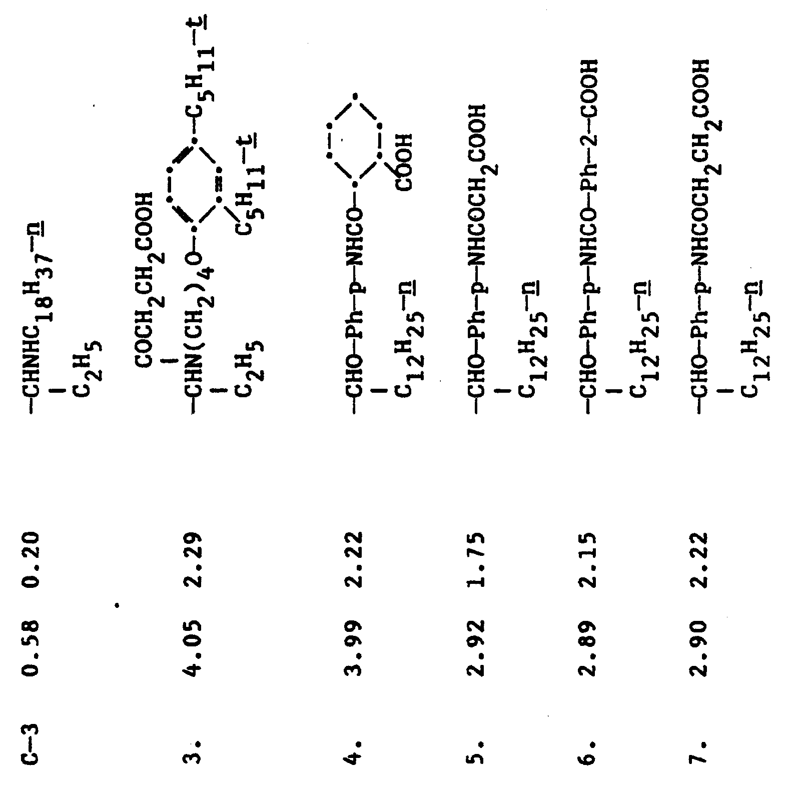

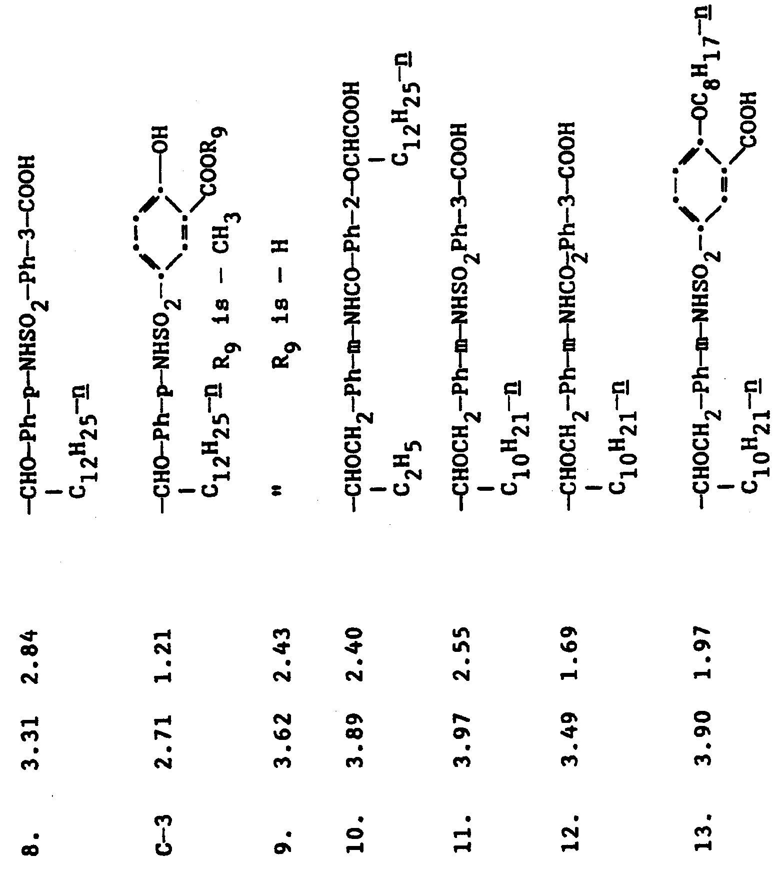

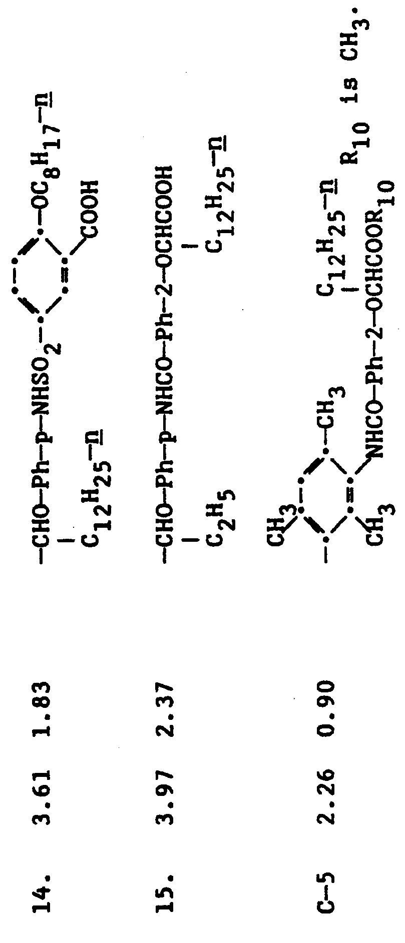

## Claims

1. A photographic silver halide material comprising a support bearing a photographic silver halide emulsion layer having in or adjacent the emulsion layer an immobile dye-forming pyrazolotriazole coupler wherein the coupler has an aliphatic or aromatic ballast group attached to the pyrazolotriazole nucleous by a secondary carbon atom containing a combination of an ether group and a carboxy group enabling increased coupler reactivity.

2. A photographic material as claimed in claim 1 wherein the pyrazolotriazole coupler is a 1 H-pyrazolo[3,2-c]-s-triazole.

3. A photographic element as in claim 1 wherein the ballast group is selected from the following groups: 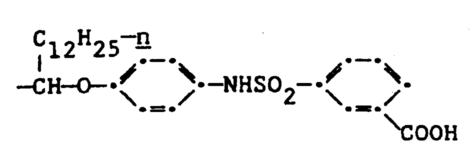 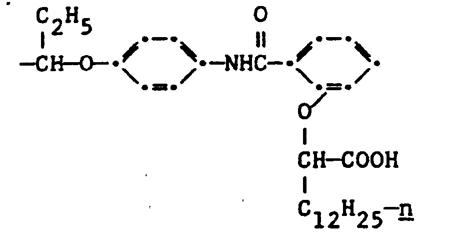 and

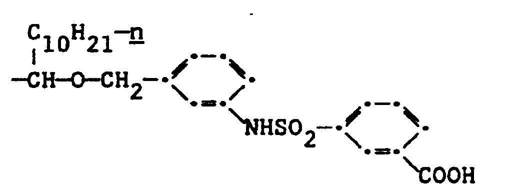

4. A photographic material as claimed in claim 1 wherein the pyrazolotriazole coupler is selected from the following couplers:

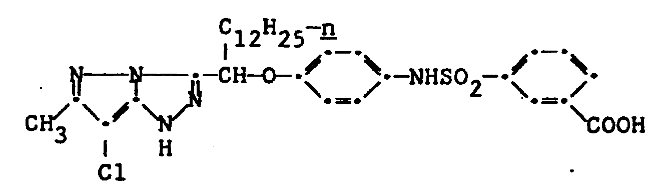

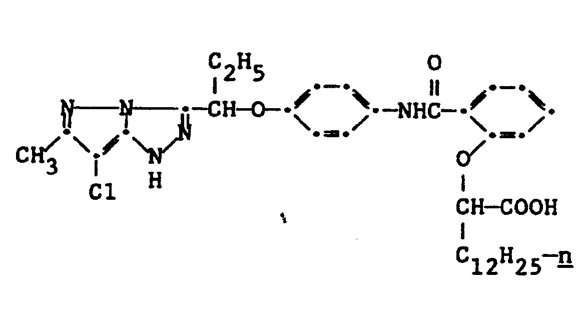

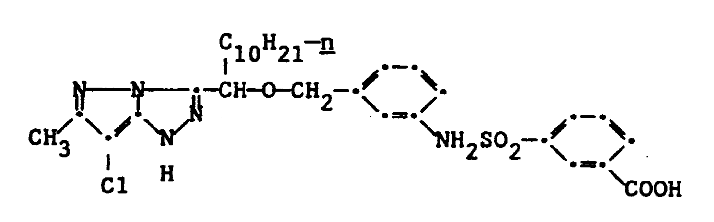

5. A process of forming a magenta dye image in an exposed photographic material as defined in any of claim 1 - 4 said process comprising developing the exposed photographic material with a silver halide color dₗ veloping agent.

6. A pyrazolotriazole coupler as defined in any of claims 1 - 4.

7. A pyrazolotriazole coupler as claimed in claim 6 wherein the ballast group is

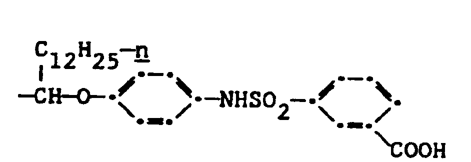

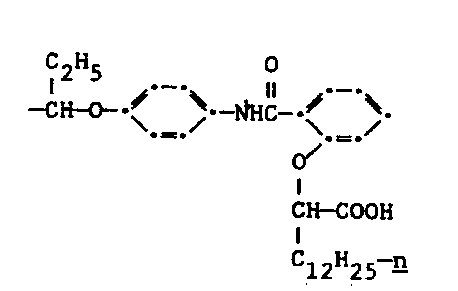

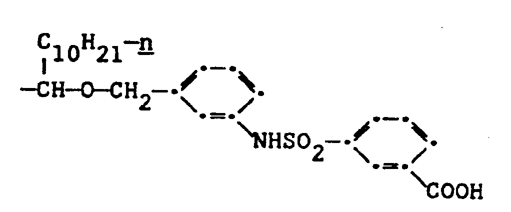

8. A pyrazolotriazole coupler that is:

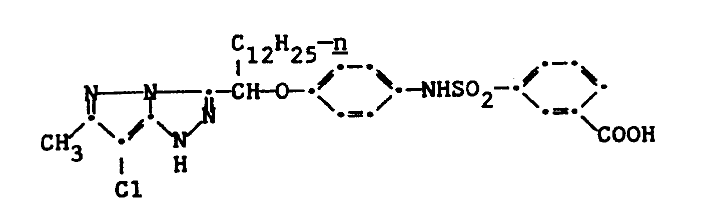

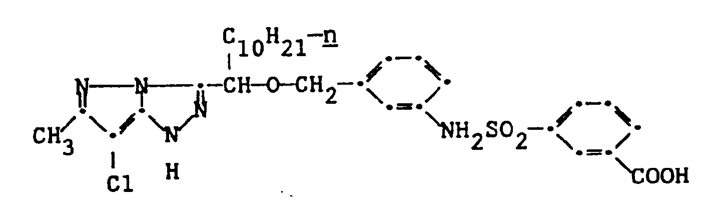

## Patentansprüche

1. Photographisches Silberhalogenidmaterial mit einem Träger, der eine photographische Silberhalogenidemulsionsschicht trägt, wobei in dieser Schicht oder einer zur Emulsionsschicht benachbarten Schicht ein einen immobilen Farbstoff bildender Pyrazolotriazol-Kuppler vorhanden ist, der eine aliphatische oder aromatische Ballastgruppe , die über ein sekundäres Kohlenstoffatom an den Pyrazolotriazolkern gebunden ist, mit einer Kombination aus einer Ethergruppe und einer Carboxygruppe, die eine erhöhte Kupplerreaktivität bewirken, aufweist.

2. Photographisches Material nach Anspruch 1, in dem der Pyrazolotriazol-Kuppler ein 1 H-Pyrazolo[3,2-c]-s-triazol ist.

3. Photographisches Element nach Anspruch 1, in dem die Ballastgruppe aus den folgenden Gruppen ausgewählt ist: 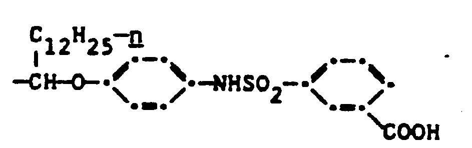 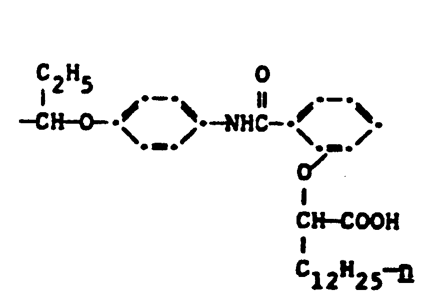 und

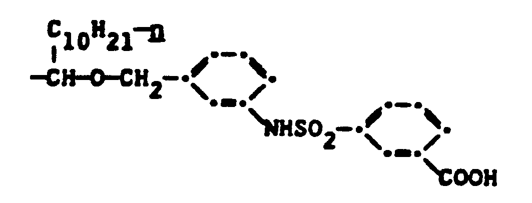

4. Photographisches Material nach Anspruch 1, in dem der Pyrazolotriazol-Kuppler aus den folgenden Kupplern ausgewählt ist:

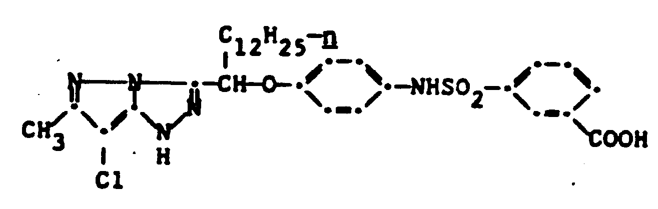

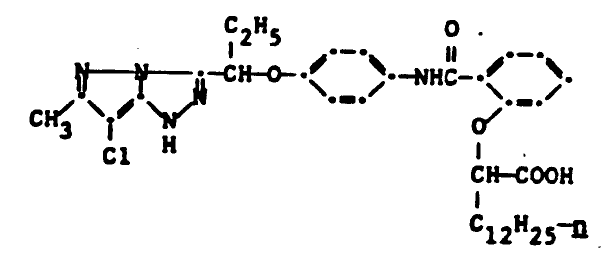

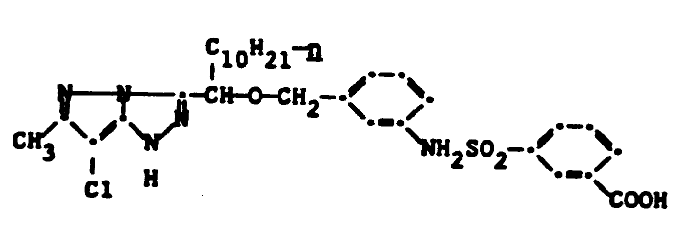

5. Verfahren zur Herstellung eines purpurroten Farbstoffbildes in einem exponierten photographischen Material nach einem der Ansprüche 1 - 4, bei dem das exponierte photographische Material mit einer Silberhalogenidentwicklerverbindung entwickelt wird.

6. Pyrazolotriazol-Kuppler nach einem der Ansprüche 1 - 4.

7. Pyrazolotriazol-Kuppler nach Anspruch 6, in dem die Ballastgruppe die folgende Formel hat:

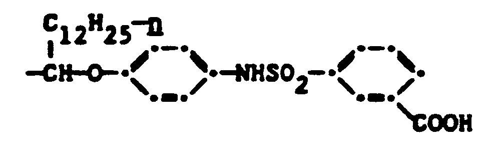

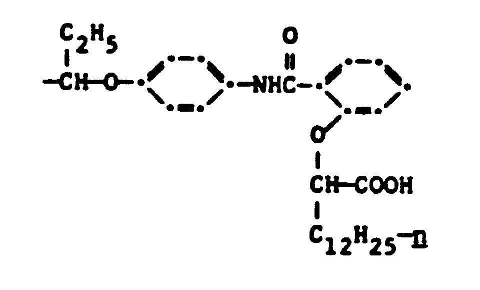

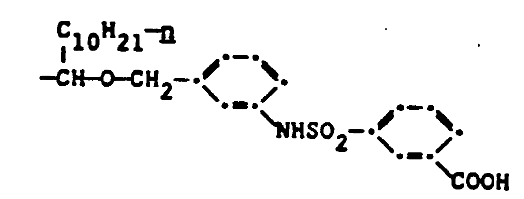

8. Pyrazolotriazol-Kuppler der folgenden Formel:

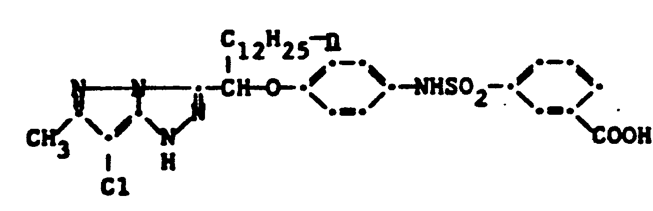

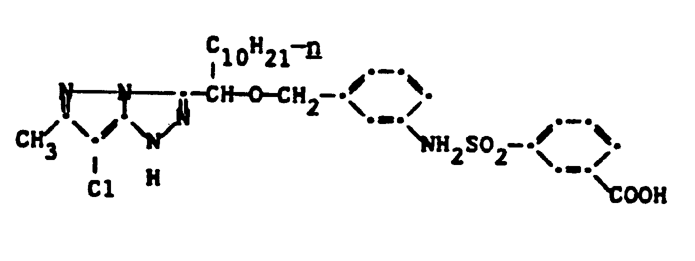

## Revendications

1. Produit photographique aux halogénures d'argent comprenant un support portant une couche d'émulsion aux halogénures d'argent photographiques, contenant dans cette couche d'émulsion ou dans une couche adjacente, un coupleur pyrazolotriazole formateur de colorant immobile dont le groupe ballast aliphatique ou aromatique est fixé sur le noyau pyrazolotriazole par un atome de carbone secondaire et contient une combinaison d'un groupe éther et d'un groupe carboxy capable d'augmenter la réactivité du coupleur.

2. Produit photographique selon la revendication 1, dans lequel le coupleur pyrazolotriazole est un 1 H-py- razolo-[3,2-c]-s-triazole.

3. Produit photographique selon la revendication 1, dans lequel le groupe ballast est choisi parmi les groupes suivants :

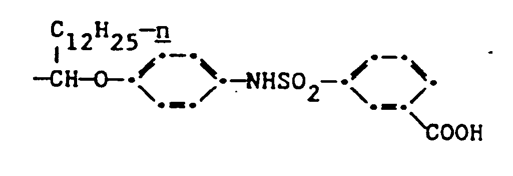

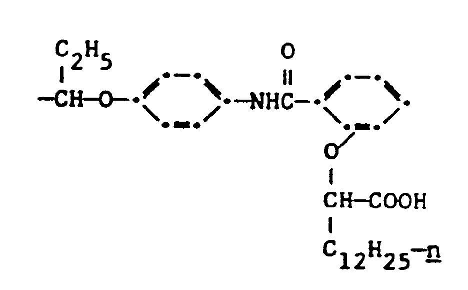

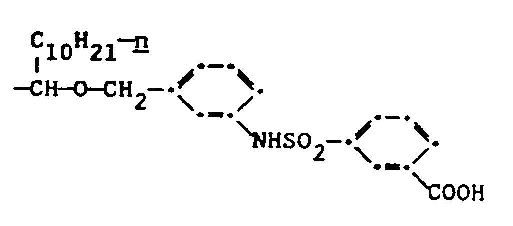

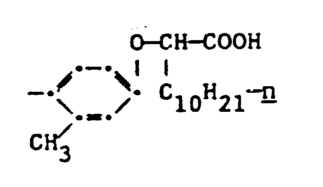

4. Produit photographique selon la revendication 1, dans lequel le coupleur pyrazolotriazole est choisi parmi les coupleurs suivants :

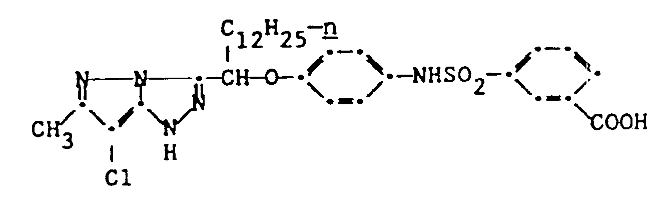

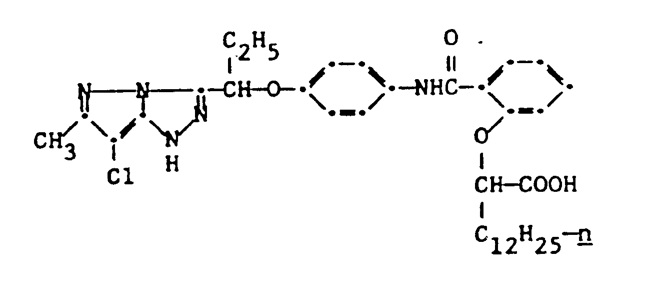

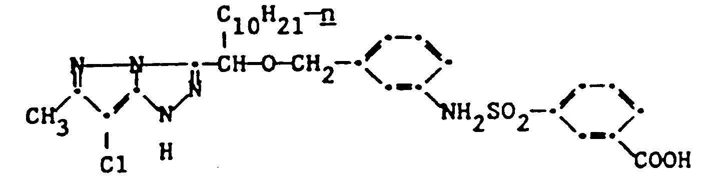

5. Procédé de formation d'une image de colorant magenta dans un produit photographique exposé tel que défini à l'une quelconque des revendications 1 à 4, consistant à développer le produit photographique exposé avec un développateur chromogène des halogènures d'argent.

6. Coupleur pyrazolotriazole tel que défini à l'une quelconque des revendications 1 à 4

7. Coupleur pyrazolotriazole selon la revendication 6, dans lequel le groupe ballast est :

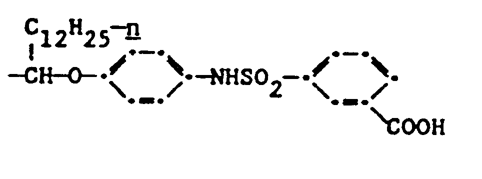

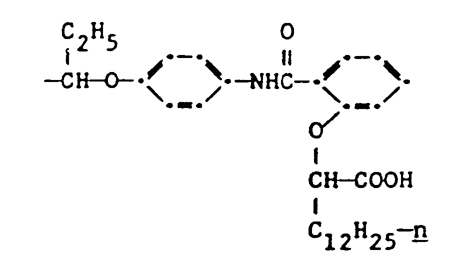

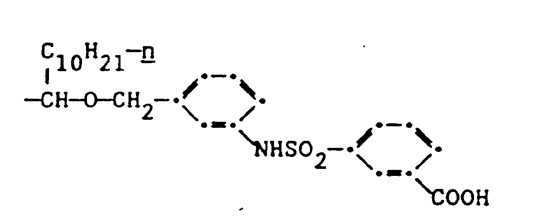

8. Coupleur pyrazolotriazole de formule :
